# EUROPÄISCHE PATENTSCHRIFT

(11) **EP 1 260 188 B1**
(45) Veröffentlichungstag und Bekanntmachung des Hinweises auf die Patenterteilung: **17.09.2014**
(21) Anmeldenummer: 01810517.1
(22) Anmeldetag: 25.05.2001
(51) Int. Cl.: A61B 17/74, A61B 17/72

(54) **Oberschenkel-Marknagel zum Einbringen am Kniegelenk**
Femoral bone nail for implantation in the knee
Clou d'os fémorale pour l'implantation dans le genou

(43) Veröffentlichungstag der Anmeldung: 27.11.2002
(73) Patentinhaber: Zimmer GmbH, 8404 Winterthur (CH)
(72) Erfinder: Bühler, Daniel W., Dr., 8304 Wallisellen (CH)
(74) Vertreter: Manitz, Finsterwald & Partner GbR

(56) Entgegenhaltungen:
- EP-A- 0 355 411
- EP-A- 0 853 923
- EP-A- 1 095 626
- FR-A- 2 718 013
- US-A- 5 472 444
- US-A- 5 480 402
- US-A- 5 779 705

## Beschreibung

Die Erfindung handelt von einem Oberschenkel-Marknagel zum Einbringen zwischen den Kondylen eines Kniegelenks mit einer Verankerung im Bereich der Nagelspitze, mit einer in der Sagittalebene vorgesehenen Krümmung, mit einem geraden Endstück, das zu seiner Befestigung Befestigungsbohrungen mit Achsen B, C aufweist, und mit den weiteren Merkmalen des Oberbegriffs des Anspruchs 1.

Derartige Oberschenkel-Marknägel werden verwendet, wenn sich eine Öffnung des Kniegelenks ergibt. Bei Unfällen kommt es immer wieder vor, dass Fragmente im Bereich der Kondylen des Kniegelenks entstehen. Die bisherigen Heilungsmöglichkeiten bestehen in am Femurknochen mit Knochenschrauben angebrachten Platten (Manual der Osteosynthese, Springer Verlag, 1992: Abb. 12.10; 12.11; 12.12), welche ein grosses Operationsfeld ergeben oder in Marknägeln, wie sie in der Patentanmeldung EP-A-0 827 717 gezeigt sind.

In der EP-A-0 827 717 sind im kondylaren Bereich mehrere zueinander parallel verlaufende Schraubverbindungen durch einen Schaft gezeigt, die mit ihren Eintritts- und eventuellen Austrittsöffnungen im Bereich des eigentlichen Gelenks liegen und Auswirkungen auf Bänder, Nerven und Blutgefässe haben können und so indirekt den Heilungsprozess erschweren können.

Diesem Umstand soll die Erfindung entgegenwirken. Sie hat die Aufgabe die Fixierung von Fragmenten im kondylaren Bereich zu verbessern und erreicht dies dadurch, dass mindestens zwei Befestigungsbohrungen mit Achsen B und C in einem Abstand kleiner 70 Millimeter vom Ende des Endstücks die Längsachse X des Endstücks kreuzen und mit ihren Achsen B und C eine Ebene, die das Ende enthält und senkrecht zur Längsachse X steht, in einem Abstand r von der X-Achse in einem Bereich 10 mm ≤ r ≤ 116 mm durchstossen, um harte Knochenzonen im Bereich der Kondylen mit Befestigungsschrauben zu fassen, und durch die weiteren Merkmale des kennzeichnenden Teils des Anspruchs 1.

Ein Marknagel mit den Merkmalen des Oberbegriffs des Anspruchs 1, allerdings für durch den Oberschenkelhals gehende Befestigungsschrauben, ist aus EP 0 853 923 A1 bekannt.

Diese Anordnung hat den Vorteil, dass in den Kondylen, die von der Längsachse des Oberschenkelknochens wie der obere Teil eines "Y" weggespreizt sind, eine Verankerung in der Spreizrichtung und in harten Knochenschichten beispielsweise in posterioren Bereichen der Kondyle erfolgen kann. Ein weiterer Vorteil liegt darin, dass nur ein Minimum an Knochenöffnungen im unmittelbaren Bereich der Gelenkflächen notwendig ist.

Vorteilhafte Weiterbildungen der Erfindung ergeben sich aus den abhängigen Ansprüchen 2 bis 11. Die nachfolgenden Zahlenangaben für Winkel beziehen sich auf ein rechtes Knie. Für ein linkes Knie muss man sich das System gespiegelt vorstellen.

Besonders günstige Anordnungen ergeben sich, wenn in einer Projektion in Richtung der Längsachse X des Endstücks die Achse B in einem Winkelbereich 35° ≤ β₂ ≤ 55° und die Achse C in einem Winkelbereich 30° ≤ β₃ ≤ 45° zur Sagittalebene verlaufen und wenn die Achse B in einen Winkel 110° ≤ α₂ ≤ 150° und die Achse C einen Winkel 125° ≤ α₃ ≤ 160° mit der Längsachse X bilden. Die zugehörigen Knochenschrauben können bei einer solchen Anordnung besonders weit in die Kondylen hineingeführt werden und diese stützen, ohne dass die Gelenkflächen und ihre unmittelbare Umgebung beeinträchtigt werden.

Wenn die Längsachse X vom Ende des Marknagels her gesehen von einer Achse A einer Querbohrung senkrecht zur Sagittalebene in einem Abstand 10 mm ≤ x₁ ≤ 18 mm, von der Achse B in einem Abstand 20 mm ≤ x₂ ≤ 42 mm und von der Achse C in einem Abstand 30 mm ≤ x₃ ≤ 70 mm gekreuzt wird, können für verschiedene Kondylengrössen die harten posterioren Knochenbereiche erreicht werden.

Mit einem Endstück, dessen Durchmesser grösser als der des restlichen Marknagels ist, lässt sich eine bessere Führungslänge für die Knochenschrauben erreichen. Gleichzeitig entsteht eine grössere radiale Abstützfläche für das Endstück im Knochen. Das Auffinden der Bohrungen in den Achsen A, B oder C erfolgt durch einen Zielbügel, der formschlüssig auf dem eingeschlagenen Marknagel befestigt ist und der Bohrführungen in Richtung der Achsen A, B, C aufweist, um mit einem kleineren Bohrerdurchmesser als dem der späteren Knochenschrauben den Knochen bis zu den Bohrungen im Marknagel anzubohren. Die Knochenschrauben können selbstschneidend sein.

Der Marknagel kann mit einem Knick zwischen Endstück und dem eigentlichen längeren Teil des Marknagels versehen sein. Ein solcher Knick erlaubt es, das Endstück im Bereich des Zielbügels gerade und in engen Toleranzen herzustellen und trotzdem der Krümmung des Oberschenkelknochens in der Sagittalebene zu folgen. Der Knick wird kleiner als 8° gehalten, um im Bereich der Krümmung des Oberschenkels zu bleiben.

Entsprechend einer üblichen Operationstechnik kann der Marknagel hohl d.h. kanuliert sein, um entlang einem Führungsdraht eingeschlagen zu werden. Er kann auch ausserhalb des Endstücks längsgeschlitzt sein, um eine grössere Elastizität zu erhalten.

Im folgenden wird die Erfindung anhand von Ausführungsbeispielen beschrieben. Es zeigen:
- Fig. 1: Schematisch die Ansicht eines erfindungsgemässen Oberschenkel-Marknagels mit einem Koordinatensystem zur Darstellung des Endstücks;
- Fig. 2: schematisch eine vergrösserte Draufsicht in Richtung der X-Achse auf das Endstück von Fig. 1;
- Fig. 3: schematisch eine Seitenansicht auf das Endstück von Fig. 2 in Richtung der Y-Achse;
- Fig. 4: schematisch eine Seitenansicht auf das Endstück von Fig. 2 senkrecht zu einer Ebene die durch die Längsachse X und durch die Achse B gegeben ist;
- Fig. 5: schematisch eine Seitenansicht auf das Endstück von Fig. 2 senkrecht zu einer Ebene die durch die Längsachse X und die Achse C gegeben ist;
- Fig. 6: schematisch eine Seitenansicht senkrecht zur Sagittalebene auf einen weiteren Oberschenkel-Marknagel, der in einem Femurknochen eingeschlagen ist; und
- Fig. 7: schematisch eine vergrösserte Ansicht eines Zielbügels der für die Endstücke der Figuren 1 und 6 verwendbar ist.

Die Figuren zeigen einen Oberschenkel-Marknagel zum Einbringen zwischen den Kondylen eines Kniegelenks mit einer Verankerung 4 im Bereich der Nagelspitze 3, mit einer in der Sagittalebene 5 verlaufenden Krümmung 6 und mit einem geraden Endstück 7, das zu seiner Befestigung Befestigungsbohrungen 12, 13 mit Achsen B, C aufweist. Mindestens zwei Befestigungsbohrungen 12, 13 mit Achsen B und C sind in einem Abstand X₂, X₃ kleiner 70 Millimeter vom Ende 8 des Endstücks 7 angebracht, deren Achsen B und C eine Ebene 10, die das Ende 8 enthält und senkrecht zur Längsachse X steht, in einem Abstand r von der X-Achse in einem Bereich 10 mm ≤ r ≤ 116 mm durchstossen, um harte Knochenzonen im Bereich der Kondylen mit Befestigungsschrauben zu fassen.

In den Figuren sind gleiche Hinweiszeichen für gleiche Funktionen verwendet.

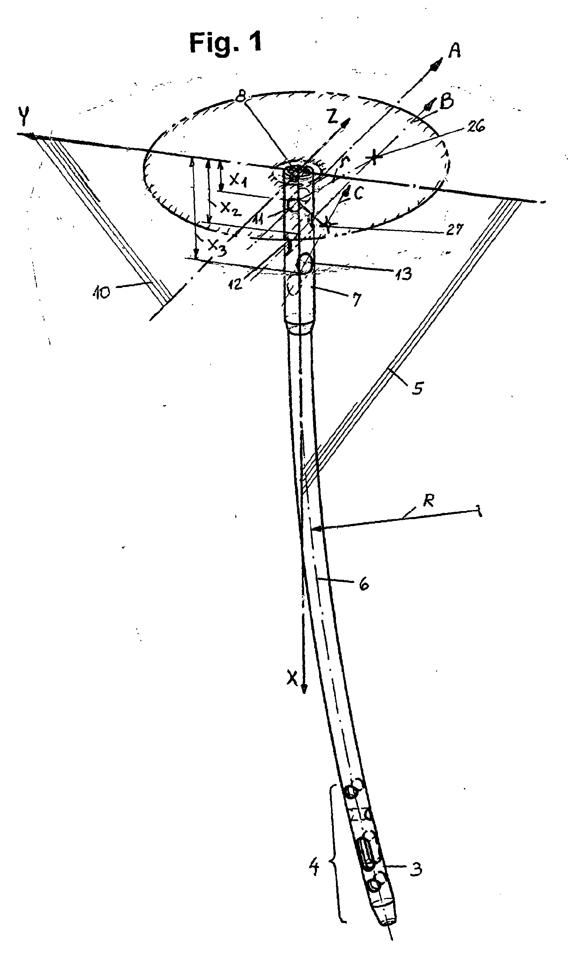

Der Marknagel der in den Figuren 1 bis 5 dargestellt ist, bezieht sich auf einen rechten Oberschenkel und besteht aus einem geraden Endstück 7, welches einen grösseren Durchmesser 18 als der Durchmesser 17 des daran anschliessenden mit einer Krümmung 6 in der Sagittalebene 5 verlaufenden Restteils aufweist. Das Koordinatensystem in Fig. 1 ist so angeordnet, dass die Längsachse X des Endstücks in proximaler Richtung verläuft, dass die zur X-Achse senkrecht verlaufende Y-Achse mit der X-Achse in der Sagittalebene 5 liegt und gegen anterior verläuft, während die Z-Achse gegen medial verläuft. Bei einem Marknagel für den linken Oberschenkel sind die Verhältnisse entsprechend gespiegelt. Der Marknagel ist hohl und hat eine durchgehende Öffnung in Längsrichtung, um ihn während dem Einschlagen an einem Führungsdraht zu führen. Das Endstück 7 ist gerade und erlaubt es den Markraum vom Knie her mit einem maschinellen Bohrer anzubohren. Dadurch, dass das Endstück einen grösseren Durchmesser 18 hat, kann der gekrümmte vordere Teil problemlos eingeschoben werden. Beispielsweise hat das Endstück 7 einen Durchmesser 18 von 18 mm und der gekrümmte Teil einen Durchmesser 17 von 10 mm. Die Krümmung 6 des vorderen Schaftteils entspricht einen Krümmungsradius R zwischen 800 mm bis 1200 mm. Der Marknagel ist an seiner Nagelspitze 3 mit einer Verankerung 4 versehen, die aus Querbohrungen für Knochenschrauben bestehen kann. Diese können auch in Form eines Langloches ausgeführt sein, um mit zulässigen Kleinstbewegungen in Längsrichtung die Kallusbildung an Bruchstellen des Knochens zu fördern.

In einem Abstand X₁ zwischen 10 und 18 mm vom Ende 8 des Endstücks 7 ist eine erste Querbohrung 11 angebracht, deren Achse A senkrecht zur X-Achse und senkrecht zur Sagittalebene 5, d.h. parallel zur Z-Achse verläuft. Senkrecht zur X-Achse verläuft eine Ebene 10, die das Ende 8 und die Achsen Y, Z enthält.

In einem Abstand X₂ zwischen 20 und 42 mm vom Ende 8 des Endstücks kreuzt eine schräg angebrachte Befestigungsbohrung 12 mit Achse B die Längsachse X und in einem Abstand X₃ zwischen 30 und 70 mm vom Ende 8 kreuzt eine schräg angebrachte Befestigungsbohrung 13 mit Achse C die Längsachse X. Beide Befestigungsbohrungen 12 und 13, sind so angebracht, dass ihre Achsen B und C die Ebene 10 an Durchstosspunkten 26 und 27 durchstossen, wobei der Abstand r dieser Durchstosspunkte zur X-Achse in einem Bereich zwischen 10 und 116 mm liegt.

In Fig. 1 ist dieser Bereich für die Durchstosspunkte 26 und 27 durch einen Kreisring in der Ebene 10 angedeutet.

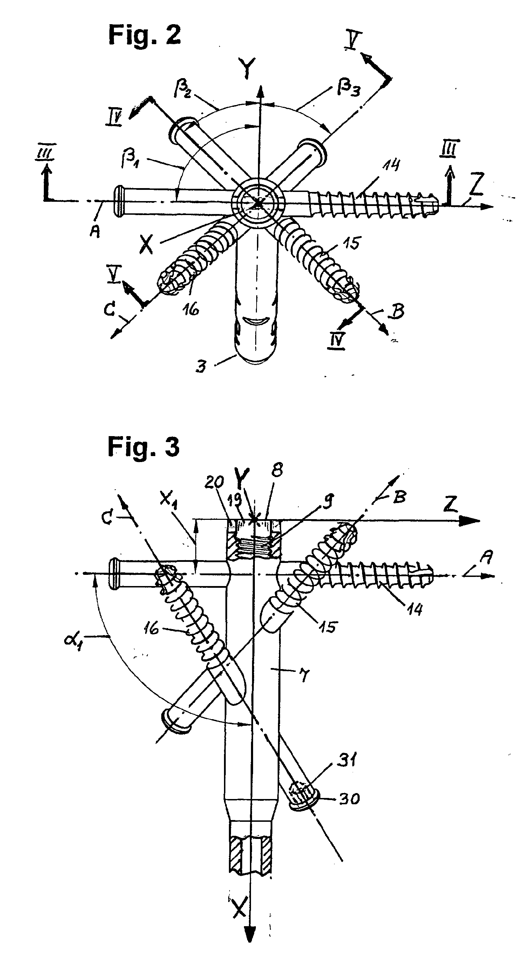

Zum besseren Verständnis der Richtungen der Achsen A, B, C sind in den Figuren 2 bis 5 die zugehörigen Befestigungsschrauben 14, 15, 16 eingezeichnet.

In Fig. 2 ergeben sich zur Y-Achse resp. zur Sagittalebene 5 für die Achse A ein Winkel β₁ von 90°, für die Achse B ein Winkel β₂ zwischen 35° und 55° und für die Achse C auf der entgegengesetzten Seite zur Achse Y ein Winkel β₃ zwischen 30° und 45°.

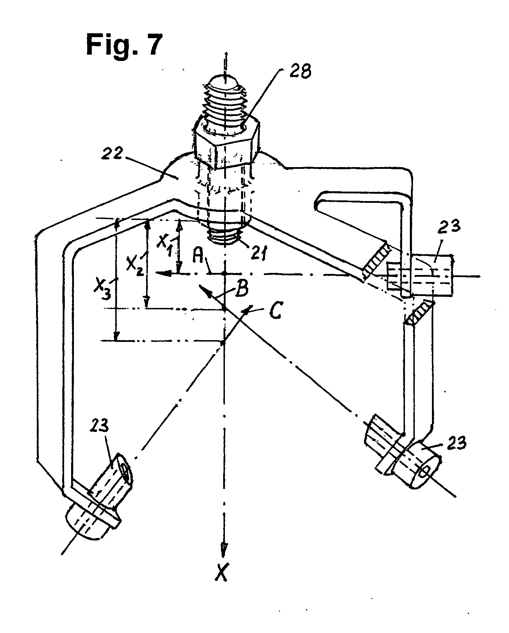

In Fig. 3 zeigt die Seitenansicht in Richtung der Y-Achse, dass die Achse A im Abstand X₁ parallel zur Z-Achse verläuft und einen Winkel α₁ von 90° zur X-Achse bildet. Im weiteren erkennt man, dass der Marknagel hohl ist und an seinem Ende 8 eine Rundführung 19 und einen Schlitz 20 sowie ein Innengewinde 9 für die formschlüssige Befestigung eines Zielbügels 22, wie er in Fig. 7 gezeigt ist, aufweist. Die Befestigungsschrauben 16 selbst besitzen an ihrem Ende einen schmalen Kragen 30 und einen Innensechskant 31 für ein Eindrehwerkzeug.

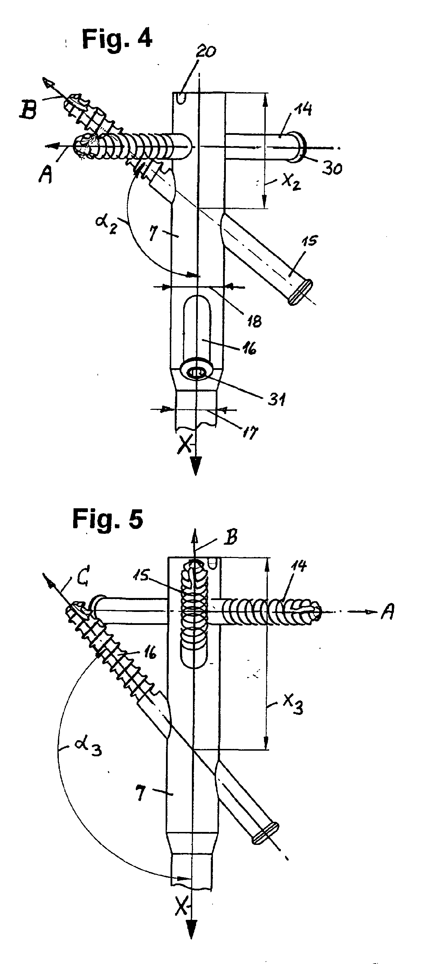

Fig. 4 zeigt analog zu Fig. 3, dass die Achse B in einem Winkel α₂ zwischen 110° und 150° zur X-Achse angeordnet ist und in einem Abstand X₂ zwischen 20 und 42 mm die X-Achse kreuzt. Ausserdem ist der grössere Durchmesser 18 des Endstücks 7 mit einem konischen Übergang zum Durchmesser 17 des vorderen Teils versehen.

In Fig. 5 kreuzt die Achse C die X-Achse in einem Abstand X₃ zwischen 30 und 70 mm und bildet zu dieser einen Winkel α₃ zwischen 30 und 45°.

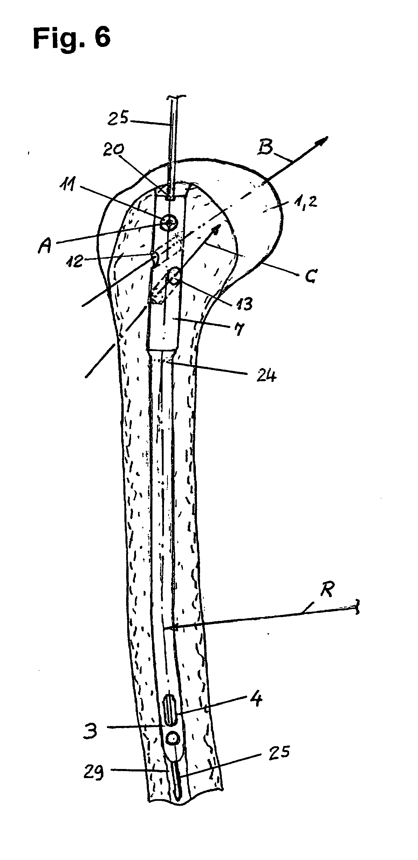

Der Marknagel kann eine Länge zwischen 480 mm und 150 mm aufweisen. In Fig. 6 ist eine gegenüber Fig. 1 kürzere Version gezeigt. Der Marknagel sitzt bereits eingeschlagen in einem Femurknochen, bei dem die Bruchgrenzen von Fragmenten nicht sichtbar sind. Ein Führungsdraht 25, entlang welchem der Marknagel eingeschlagen wurde, ist noch nicht entfernt. Die beiden Achsen B, C sind in die Kondylen 1, 2 gegen posterior gerichtet. Der Marknagel weist in der Sagittalebene ebenfalls eine Krümmung mit Krümmungsradius R und zusätzlich einen Knick 24 am Übergang zum geraden Endstück 7 auf. An der Nagelspitze 3 sind zwei Querbohrungen, davon eine als Langloch, für eine Verankerung 4 angebracht. Entsprechend den gezeigten Richtungen der Achsen B und C werden die Befestigungsschrauben eingeschraubt, wobei die gegen aussen tragenden Flächen der Kondylen resp. von Kondylenfragmenten unversehrt bleiben.

In Fig. 7 ist eine passende Zentriervorrichtung mit einem Zielbügel 22 gezeigt, der am Marknagel formschlüssig mit einer Verbindungsschraube 28 befestigt werden kann, welche mit ihrem Gewinde 21 am entsprechenden Innengewinde 9 (Fig. 3) eingreift. Der Zentrierbügel wird an einer Rundführung 19 innenzentriert, am Ende 8 in der Y-Z-Ebene plan ausgerichtet und rastet mit einer Schulter in einer vorgesehenen Winkellage formschlüssig im Schlitz 20 ein. Die Verbindungsschraube 28 ist mit einem Aussensechskant versehen, um den Zielbügel 22 anzuschrauben und an einem nach oben fortgesetzten Gewinde einen Gleithammer anzuschrauben. Die Lage der Achsen A, B, C die in den Abständen X₁, X₂, X₃ die X-Achse kreuzen ist mit Bohrführungen 23 wiederholt, um am eingeschlagenen Marknagel den Knochen mindestens bis zu den Befestigungsbohrungen 11, 12, 13 im Endstück 7 mit einem geführten Bohrwerkzeug anzubohren. Die eigentlichen Bügel, welche die Verbindung zu den Bohrführungen 23 bilden, müssen genügend weit von der X-Achse wegstehen, damit sie nicht mit den Kondylen kollidieren. Andererseits sollten die Bohrführungen 23 möglichst nah an den zu bohrenden Knochen herangeführt sein, um schräg zur Knochenoberfläche angesetzte Bohrwerkzeuge ausreichend zu führen. Aus diesem Grund ist es vorteilhaft zunächst die Bohrung in Richtung der Achse A vorzunehmen und über diese Bohrung eine provisorische Fixierung zum Beispiel durch Stehenlassen vom Bohrwerkzeug vorzunehmen, bevor man in den Achsrichtungen B und C bohrt.

## Patentansprüche

1. Oberschenkel-Marknagel zum Einbringen zwischen den Kondylen (1, 2) eines Kniegelenks mit einer Verankerung (4) im Bereich der Nagelspitze (3), mit einer in der Sagittalebene (5) vorgesehenen Krümmung (6) und mit einem geraden Endstück (7), das zu seiner Befestigung Befestigungsbohrungen (12, 13) mit Achsen B, C aufweist, wobei mindestens zwei Befestigungsbohrungen (12, 13) mit Achsen B und C in einem Abstand kleiner 70 Millimeter vom Ende (8) des Endstücks (7) die Längsachse X des Endstücks (7) kreuzen und mit ihren Achsen B und C eine Ebene (10), die das Ende (8) enthält und senkrecht zur Längsachse X steht, in einem Abstand r von der Längsachse X in einem Bereich 10 mm ≤ r ≤ 116 mm durchstossen, um harte Knochenzonen im Bereich der Kondylen (1, 2) mit Befestigungsschrauben (15, 16) zu fassen,
**dadurch gekennzeichnet,**
**dass** in einer Projektion in Richtung der Längsachse X des Endstücks (7) die beiden in Richtung des Endes (8) des Endstücks (7) gerichteten Halbachsen B, C jeweils einen Winkel β₂, β₃ zur Sagittalebene (5) einschließen und die beiden Winkel β₂, β₃ auf entgegengesetzten Seiten der Sagittalebene (5) liegen und
**dass** 30° ≤ β₃ ≤45° gilt.

2. Oberschenkel-Marknagel nach Anspruch 1,
**dadurch gekennzeichnet,**
**dass** in einer Projektion in Richtung der Längsachse X des Endstücks (7) die Achse B in einem Winkelbereich 35° ≤ β₂ ≤ 55° zur Sagittalebene (5) verläuft.

3. Oberschenkel-Marknagel nach einem der Ansprüche 1 bis 2,
**dadurch gekennzeichnet,**
**dass** die Achse B zur Längsachse X einen Winkel 110 ≤ α₂ ≤ 150° bildet.

4. Oberschenkel-Marknagel nach einem der Ansprüche 1 bis 3,
**dadurch gekennzeichnet,**
**dass** die Achse C zur Längsachse X einen Winkel 125 ≤ α₃ ≤ 160° bildet.

5. Oberschenkel-Marknagel nach einem der Ansprüche 1 bis 4,
**dadurch gekennzeichnet,**
**dass** eine zusätzliche Querbohrung (11) mit Achse A die Längsachse X des Endstücks (7) in einem Abstand X₁ von deren Ende (8) kreuzt und senkrecht zur Sagittalebene (5) steht.

6. Oberschenkel-Marknagel nach Anspruch 5,
**dadurch gekennzeichnet,**
**dass** die Längsachse X vom Ende (8) aus von der Achse A in einem Abstand 10 mm ≤ X₁ ≤ 18 mm, von der Achse B in einem Abstand 20 mm ≤ X₂ ≤ 42 mm und von der Achse C in einem Abstand 30 mm ≤ X₃ ≤ 70 mm durchkreuzt ist.

7. Oberschenkel-Marknagel nach einem der Ansprüche 1 bis 6,
**dadurch gekennzeichnet,**
**dass** das Endstück (7) einen grösseren Durchmesser (18) als der gekrümmte Teil aufweist.

8. Oberschenkel-Marknagel nach eine der Ansprüche 1 bis 7,
**dadurch gekennzeichnet,**
**dass** das Endstück (7) eine formschlüssige Verbindung (19, 20, 21) für einen Zielbügel (22) mit Bohrführungen in Richtung der Achsen A, B, und C aufweist.

9. Oberschenkel-Marknagel nach einem der Ansprüche 1 bis 8,
**dadurch gekennzeichnet,**
**dass** der Übergang vom Endstück (7) zum weiteren Marknagel mit einem Knick (24) in der Sagittalebene von weniger als 8° Richtungsänderung versehen ist.

10. Oberschenkel-Marknagel nach einem der Ansprüche 1 bis 9,
**dadurch gekennzeichnet,**
**dass** der Marknagel für einen eventuellen Führungsdraht kanuliert ist.

11. Oberschenkel-Marknagel nach einem der Ansprüche 1 bis 10,
**dadurch gekennzeichnet,**
**dass** der Marknagel ausserhalb des Endstücks (7) einen Längsschlitzt aufweist.

## Claims

1. A femur medullary nail for insertion between the condyles (1, 2) of a knee joint having an anchoring (4) in the region of the nail tip (3), having a curvature (6) provided in the sagittal plane (5) and having a straight end piece (7) which has fastening bores (12, 13) with axes B, C for its fastening, wherein at least two fastening bores (12, 13) cross the longitudinal axis X of the end piece (7) with axes B and C at a spacing less than 70 millimetres from the end (8) of the end piece (7), and with their axes B and C penetrating a plane (10) which includes the end (8) and is perpendicular to the longitudinal axis X at a distance r from the longitudinal axis X in a region of 10 mm ≤ r ≤ 116 mm in order to grip hard bone zones with fastening screws (15, 16) in the region of the condyles (1, 2),
**characterised in that**,
in a projection in the direction of the longitudinal axis X of the end piece (7), the two half axes B, C directed in the direction of the end (8) of the end piece (7) each include an angle β₂, β₃ with the sagittal plane (5) and the two β₂, β₃ angles are disposed on opposite sides of the sagittal plane (5); and **in that** 30° ≤ β₃ ≤ 45° applies.

2. A femur medullary nail in accordance with claim 1,
**characterised in that**,
in a projection in the direction of the longitudinal axis X of the end piece (7), the axis B extends in an angle region 35° ≤ β₂ ≤ 55° to the sagittal plane (5).

3. A femur medullary nail in accordance with claim 1 or claim 2,
**characterised in that**,
the axis B forms an angle of 110 ≤ α₂ ≤ 150° relative to the longitudinal axis X.

4. A femur medullary nail in accordance with any one of the claims 1 to 3,
**characterised in that**
the axis C forms an angle of 125 ≤ α₃ ≤ 160° relative to the longitudinal axis X.

5. A femur medullary nail in accordance with any one of the claims 1 to 4,
**characterised in that**
an additional transverse bore (11) crosses the longitudinal axis X of the end piece (7) with an axis A at a spacing X₁ from its end (8) and is perpendicular to the sagittal plane (5).

6. A femur medullary nail in accordance with claim 5,
**characterised in that**
the longitudinal axis X is crossed starting from the end (8) by the axis A at a spacing of 10 mm ≤ X₁ ≤ 18 mm, by the axis B at a spacing of 20 mm ≤ X₂ ≤ 42 mm and by the axis C at a spacing of 30 mm ≤ X₃ ≤ 70 mm.

7. A femur medullary nail in accordance with any one of the claims 1 to 6,
**characterised in that**
the end piece (7) has a greater diameter (18) than the curved part.

8. A femur medullary nail in accordance with any one of the claims 1 to 7,
**characterised in that**
the end piece (7) has a form-locked connection (19, 20, 21) for a target wire (22) with bore guides in the direction of the axes A, B and C.

9. A femur medullary nail in accordance with any one of the claims 1 to 8,
**characterised in that**
the transition from the end piece (7) to the further medullary nail is provided with a kink (24) in the sagittal plane of less than 8° of change of direction.

10. A femur medullary nail in accordance with any one of the claims 1 to 9,
**characterised in that**
the medullary nail is cannulated for a possible guide wire.

11. A femur medullary nail in accordance with any one of the claims 1 to 10,
**characterised in that**
the medullary nail has an elongate slot outside the end piece (7).

## Revendications

1. Clou médullaire pour fémur destiné à être introduit entre les condyles (1, 2) d'une articulation du genou, comprenant un ancrage (4) dans la région de la pointe du clou (3), comportant une courbure (6) prévue dans le plan sagittal (5) et avec une pièce terminale droite (7) qui comporte pour sa fixation des perçages de fixation (12, 13) avec des axes B, C, dans lequel au moins deux perçages de fixation (12, 13) avec les axes B et C croisent l'axe longitudinal X de la pièce terminale (7) à une distance inférieure à 70 mm depuis l'extrémité (8) de la pièce terminale (7) et traversent avec leurs axes B et C un plan (10), qui contient l'extrémité (8) et qui se dresse perpendiculairement à l'axe longitudinal X, à une distance r de l'axe longitudinal dans une plage telle que 10 mm ≤ r ≤ 116 mm, afin de saisir des zones osseuses dures dans la région des condyles (1, 2) avec des vis de fixation (15, 16), **caractérisé en ce que**, dans une projection en direction de l'axe longitudinal X de la pièce terminale (7), les deux demi-axes B, C orientés en direction de l'extrémité (8) de la pièce terminale (7) définissent respectivement un angle β2, β3 par rapport au plan sagittal (5) et les deux angles (β2, β3) sont situés sur les côtés opposés du plan sagittal (5), et
**en ce que**
30° ≤ β3 ≤ 45°.

2. Clou médullaire pour fémur selon la revendication 1,
**caractérisé en ce que**, dans une projection en direction de l'axe longitudinal X de la pièce terminale (7), l'axe B s'étend dans une plage angulaire telle que 35° ≤ β2 ≤ 55° par rapport au plan sagittal (5).

3. Clou médullaire pour fémur selon l'une des revendications 1 et 2,
**caractérisé en ce que** l'axe (B) forme par rapport à l'axe longitudinal X un angle tel que 110° ≤ α2 ≤ 150°.

4. Clou médullaire pour fémur selon l'une des revendications 1 à 3,
**caractérisé en ce que** l'axe C forme par rapport à l'axe longitudinal X un angle tel que 125° ≤ α3 ≤ 160°.

5. Clou médullaire pour fémur selon l'une des revendications 1 à 4,
**caractérisé en ce qu'**un perçage transversal supplémentaire (11) avec un axe A croise l'axe longitudinal (X) de la pièce terminale (7) à une distance (X1) depuis son extrémité (8) et se dresse perpendiculairement au plan sagittal (5).

6. Clou médullaire pour fémur selon la revendication 5,
**caractérisé en ce que**, partant de l'extrémité (8), l'axe longitudinal X est croisé par l'axe A à une distance telle que 10 mm ≤ X 1 ≤ 18 mm, par l'axe B à une distance telle que 20 mm ≤ X2 ≤ 42 mm, et par l'axe C à une distance telle que 30 mm ≤ X3 ≤ 70 mm.

7. Clou médullaire pour fémur selon l'une des revendications 1 à 6,
**caractérisé en ce que** la pièce terminale (7) présente un diamètre plus grand (18) que la partie incurvée.

8. Clou médullaire pour fémur selon l'une des revendications 1 à 7,
**caractérisé en ce que** la pièce terminale (7) comporte une liaison à coopération de formes (19, 20, 21) pour un étrier-cible (22) avec des guidages de perçage en direction des axes A, B et C.

9. Clou médullaire pour fémur selon l'une des revendications 1 à 8,
**caractérisé en ce que** la transition depuis la pièce terminale (7) vers le reste du clou médullaire est dotée d'une pliure (24) dans le plan sagittal avec une modification de direction inférieure à 8°.

10. Clou médullaire pour fémur selon l'une des revendications 1 à 9,
**caractérisé en ce que** le clou médullaire présente une canule pour un fil de guidage éventuel.

11. Clou médullaire pour fémur selon l'une des revendications 1 à 10,
**caractérisé en ce que** le clou médullaire présente une fente longitudinale à l'extérieur de la pièce terminale (7).
